Europäisches Patentamt

European Patent Office

Office européen des brevets

(11) Publication number: **0 552 571 A1**

## EUROPEAN PATENT APPLICATION

(21) Application number: 92311874.9

(22) Date of filing: 31.12.92

(51) Int. Cl.5: **C12N 1/06**, //C12N15/10, C12Q1/68

(30) Priority: **09.01.92 US 819355**

(43) Date of publication of application: **28.07.93 Bulletin 93/30**

(84) Designated Contracting States: **DE ES FR GB IT SE**

(71) Applicant: **Becton Dickinson and Company One Becton Drive Franklin Lakes New Jersey 07417-1880(US)**

(72) Inventor: **Dey, Margaret Sigler 7920 Secluded Acres Road Apex, North Carolina 27502(US)** Inventor: **Keating, William Edward 500 Woodcroft Parkway No. 18-A, Durham, North Carolina 27713(US)** Inventor: **Siddiqi, Salman ul-Haq 15 Glencoe Manor Court Sparks, Maryland 21152(US)** Inventor: **Down, James Arthur 101 Charter Oaks Circle Cary, North Carolina 27511(US)**

(74) Representative: **Ruffles, Graham Keith et al MARKS & CLERK 57-60 Lincoln's Inn Fields London WC2A 3LS (GB)**

(54) **Release of intracellular components.**

(57) The invention provides a sample processing protocol which comprises:
a) adding a disinfectant to a sample, and
b) lysing microorganisms present in (a).

The invention provides a sample processing protocol that is rapid, simple, and provides intracellular components capable of detection, amplification, and the like. In addition, the sample is rendered safe for handing.

## Field of the Invention

The invention is in the field of molecular biology. More particularly, the invention is in the area of sample preparation.

## Background of the Invention

Intracellular components are essential for a variety of reasons. Intracellular components such as DNA, RNA, proteins, proteoglycans, carbohydrates, and lipids are useful for a variety of detection events.

To obtain intracellular components the cell of interest must be opened. Methods to obtain intracellular components include the use of sonication, pH extremes, detergents, chaotropes, solvents, grinding, cavitation, and osmotic shock.

Known processes for obtaining intracellular components include U.S. patent 4,830,969 which discloses a process for isolation of nucleic acids involving lysis buffer, heating, and purification. EPO 393,744 which discloses methods for detecting nucleic acid from blood or peripheral blood mononuclear cell (PBMC) fractions which involves heating the fractions at or near the boiling point of water, U.S. patent 4,923,978 which discloses a process for purifying nucleic acid which involves passing a solution of DNA and protein over hydroxylated support to bind protein, U.S. patent 4,908,318 which discloses a nucleic acid extraction involving buffy coat, protease, and perchlorate and isopropanol precipitation of DNA, U.S. patent 4,900,677 which discloses a process for isolation of high molecular weight DNA involving lysis with detergent, RNA degradation , protein denaturation with chaotropes, and DNA dialyzed and concentrated, and A. Brisson-Noel et al., The Lancet Nov 4:1069 (1989) which discloses a procedure for diagnosis of Mycobacterium tuberculosis which uses sodium hydrochloride, sodium chloride, and sodium dodecylsulphate (SDS)

It is desirable and advantageous to have a process for obtaining intracellular components which is rapid and simple. In addition, the intracellular components should be capable of detection, amplification, and the like.

## SUMMARY OF THE INVENTION

The invention provides a sample processing protocol that is rapid and simple, in addition to providing intracellular components capable of detection, amplification, and the like.

The process of the invention comprises:

a) adding a disinfectant to a sample, and

b) lysing microorganisms in (a).

The process provides several advantages including simplicity, rapidity, and safety. Not only are intracellular components liberated by practicing the process of the invention, but the sample is also rendered safe for handling by the addition of a disinfectant. Safety is a major concern when handling samples suspected of containing pathogenic organisms. In addition, the process of the invention is applicable to all microorganisms, ranging from the easily lysed microorganisms to the most difficult.

As used in this document, "Sample" refers to the assimilation of any substance from any biological source. Samples typically refers to a fluid, tissue or serum and the like obtained from blood, mucus, sputum and the like, from a human or veterinary patient. "Lysis effective amount" refers to that necessary to liberate the desired intracellular component from a sample. "Lysing reagent" refers to the spectrum of means capable of liberating a desired intracellular component from a sample.

## DETAILED DESCRIPTION OF THE INVENTION

The organism from which the intracellular components are to be obtained is not crucial to the invention. The organism can be from culture or a clinical sample. Samples can be unpurified biological samples like sputum, feces, tissue, blood, serum, other body fluids and the like. Bacteria obtained from the various sources are typically cultured, which is very time consuming, reaching three to six weeks culture time (especially in the case of Mycobacteria). However, by practicing the method of the invention, in conjunction with a sensitive detection method such as polymerase chain reaction (PCR), the need to culture can be eliminated. If culturing is not desired, the cells are generally first isolated from the source by conventional sample processing methods then usually pelleted by centrifugation and put into a cell suspension. The ability to use the process of the invention with a clinical sample is particularly advantageous.

All disinfectants are by definition bacteriocidal. However, not all disinfectants produce sufficient bacterial lysis that is compatible with subsequent DNA amplifications and/or detection. The addition of a lysis-effective disinfectant to a sample enables both the lysis of the organisms and renders the sample safe (i.e. non pathogenic). Otherwise, a sample may be rendered safe by disinfectant treatment and lysis can otherwise be obtained with a variety of lytic agents comprising enzymes and the like.

If the sample is a sputum sample, it is generally liquified by a variety of procedures prior to adding disinfectant. For example, one suitable protocol for liquification of sputum samples can be found in Kubica et al., American Review of Respiratory Diseases **87**:775 (1963), incorporated herein by reference. After liquification, the sample is usually neutralized, then separated to obtain a pellet fraction, which contains the desired organism. To the pellet fraction is added a disinfectant. If the sample does not need to be liquified, disinfectant may be added immediately.

The organism from which intracellular components are desired can also be subjected to a lysis effective amount of heat (i.e., lytic agent) in the range of about 50C to about 110C. The heat range for a particular organism is readily obtainable by sampling a portion of the source to be lysed and examining for signs of lysis (e.g., detection of intracellular components). The heat will lyse the organism with subsequent release of intracellular components. The use of heat can be effective in lysing as well as effective in rendering a sample non-pathogenic. The only limitation on the use of heating is that the particular intracellular component of interest not be susceptible to destruction by the heat. Therefore, any intracellular component that is not destroyed by the heat employed to release the components can be obtained by using the process of the invention. A variety of means for heating with the process of the invention are available. Heating means include water baths, microwaves, ovens, and the like.

The heating time required for obtaining intracellular components ranges from about two minutes to about twenty. As with the heating temperature, optimization of heating time is readily found by sampling a portion of the source to be lysed and examining for signs of lysis (e.g., detection of intracellular components), depending on the source from which the intracellular components is to be obtained.

The organisms are collected, typically by centrifugation, in the form of a pellet, then liquified, if necessary, prior to lysis. Disinfectant is added at any point during collection of organisms or after liquification. A brief incubation time may be warranted for disinfection to occur, as according to manufacturer's specifications. If required, disinfectant might be removed by centrifuging the organisms and lysis reagents added. The treated sample can then be used in protocols for separating the DNA with subsequent elution and isolation of DNA. DNA purification procedures include Celite purification procedures as disclosed in Volgelstein and Gillespie, Proc. Nat. Acac. Sci. U.S.A. **76**:615 (1979), Willis et al., Biotechniques **9**:92 (1990), and McCormick, Analytical Biochemistry **181**:66 (1989), all incorporated herein by reference. When the DNA is obtained in its desired form and purity, subsequent amplification protocols, detection protocols, and the like can be employed.

For example, typical purification steps for obtaining DNA from a lysed sample include organic extractions such as phenol/chloroform extractions.

The process of the invention is particularly beneficial for obtaining DNA or RNA from an organism. When employing heat for lysis, the process of the invention allows DNA and RNA to be liberated from organisms in single stranded form. Generally, lysis procedures for obtaining DNA provide the DNA, which form is then subjected to extra steps to obtain single stranded DNA for subsequent use. Thus, the process of the invention provides DNA in a readily useable form, single stranded or double stranded, for subsequent use.

Suitable amplification procedures include polymerase chain reaction (PCR), PCR Technology, H. A. Erlich, Ed. (Stockton Press, New York, NY, 1989), transcription-based amplification system (TAS), Proc. Natl. Acad. Sci. USA **86**:1173 (1989), ligation amplification reaction (LAR), Genomics **4**:560 (1989), ligase based amplification system (LAS), Gene **89**:117 (1990), and Q B replicase, Infect. Dis **162**:13 (1990).

Identification and location of intracellular components and microorganisms can take place by means of a variety of identifying agents. Identifying agents refers to those agents suitable for identifying microorganisms and components which include nucleic acid probes including deoxyribonucleic acid and ribonucleic acid, and the like.

The presence of a microorganism can then be detected by a variety of means, depending on the marker (e.g., signal to be detected) chosen for use with the identifying agent. The means for identification of the presence of a microorganism or component is usually dictated by the identifying agent employed. For example, nucleic acid probes (e.g., specific for a particular species) are typically labeled with $^{125}$I, $^{32}$P, fluorescent markers, and the like. The marker is then detected, which detection is an indication that the particular species or component is present. Other means for detection include blot hybridization analysis, electrophoretic gel visualization, and the like.

The reagents of this invention can be conveniently provided in the form of a kit. Such a kit can further comprise at least one type of identifying agent and a lysis effective amount of disinfectant. Specific kits

EP 0 552 571 A1

comprise identifying agents for a particular species or specific intracellular components. Specific kits also comprise particular identifying means such as nucleic acid probes or antibodies. And, the means by which the identifying agent is detected can also be specific, for example, the agent can be designed for fluorescence, radioactive, and chemiluminescence detection and, if necessary, depending on sample requirements, liquification agents, isolation agents, and the like can be included in the kit. Further embodiments of the kits comprise lysis effective amounts of reagents such as achromopeptidase and the like. The process of the invention is not limited to use with any particular organism. Organisms such as Mycobacteria, which are considered extremely difficult to obtain intracellular components from, are suitable for use with the process of the invention. Other organisms include, but are not limited to, those from the family Acetobacteraceae, Legionellaceae, Neisseriacea, Flavobacterium, Enterobacteriaceae, Vibrionaceae, Pasteurellaceae, Bacteroidaceae, Veillonellaceae, Rickettsiaceae, Bartonellaceae, Anaplasmatacae, Chlamydiaceae, Mycoplasmataceae, Spirochaetaceae, and Pseudomonadaceae.

Important mycobacteria that can be lysed by practicing the present invention include Mycobacteria, such as M. avium, M. gordonae, M. tuberculosis, M. kansasii, M. fortuitum, M.chelonae, M. bovis, M. scrofulaceum, M. paratuberculosis, M. marinum, M. simiae, M. szulgai, M. intracellulare, M. xenopi, M. ulcerans, M. leprae, M. lepraemurium, M. smegmatis, M. flavescens, M. terrae, M. nonchromogenicum, M. malmoense, M. asiaticum, M. vaccae, M. gastri, M. triviale, M. haemophilum, M. africanum, M. thermoresistable, and M. phlei. Several of the Mycobacteria are pathogenic. For example, M. tuberculosis, which already infects two billion people and infects an additional seven to nine million people each year, is an important Mycobacteria from an epidemiologic and clinical viewpoint In addition, M. avium, M. bovis, M. intracellularae, M. africanum, M. leprae, M. chelonae, M. paratuberculosis, and M. marinum, are also significant from an epidemiological and clinical viewpoint.

The following examples illustrate the specific embodiments of the invention described in this document. As would be apparent to skilled artisans, various changes and modifications are possible and are contemplated within the scope of the invention described.

EXPLANATION OF THE EXAMPLES

The experiments which follow constitute a logical progression in which it is proven for M. tuberculosis that the components of the invention (i.e. sample processing, disinfectant treatment, lysis of the organisms and subsequent detection of the released DNA) are compatible.

Example 1     This example demonstrates sensitive amplification of M. tuberculosis DNA by polymerase chain reaction.

Example 2     This example demonstrates that sample liquification by N-acetyl cysteine is compatible with PCR amplification of M. tuberculosis DNA.

Example 3     This example tests enzymes for ability to lyse Mycobacteria.

Example 4     This example demonstrates that the enzymatic treatments tested in example 3 released from M. tuberculosis DNA that was hybridizable to a radioactive probe.

Example 5     This example demonstrates that the optimal enzymatic treatment as determined in examples 3 and 4 is compatible with the sample processing method.

Example 6     This example demonstrates that the methods of sample processing as determined in example 2, mycobacterial lysis as determined in examples 3 and 4 and disinfection can be combined into a single method.

Example 7     This example demonstrates that by the process of the invention as outlined in example 6, M. tuberculosis DNA can be amplified and detected by PCR.

EXAMPLE 1

Explanation of experiments

These experiments demonstrate polymerase chain reaction (PCR) amplification of M. tuberculosis (M. tb) targets from clinical samples. Target sensitivity in the range of 10 organisms is demonstrated.

Purpose:

To determine the effect of pre-treatment of an SK4.3 (plasmid containing the IS6110 sequence (Eisenach et al., Nucleic Acid Research 18:188 (1990) and Gene Bank Accession No. M298-99) spiked human clinical sample with various enzymes before DNA purification. To examine how these treatments

4

effect the recovery and purification of both the human $\beta$-actin target and the SK4.3 plasmid DNA target.

Materials:

Two clinical samples (cervical swabs)
Sterile Milli Q $H_2O$
Achromopeptidase (Sigma, St. Louis, Mo., A-7550 Lot #127F-68391)
Mutanolysin (Sigma M-9901 Lot #98F-5822)
Trypsin (Sigma T-1005 Lot #118F-8065)
Lyticase (Sigma L-8012 Lot #77F-6832)
Lysing Enzymes (A) (Sigma L-8757 from Rhizocetoria Solani, Lot #46F-0283)
Lysing Enzymes (B) (Sigma L-3768 from Aspergillus species Lot # 59F-0141)
Proteinase K ($\mu$g/ml Sigma P-8049 Lot #68F-6839)

Buffers:

100mM Trizma 9.0 + 10mM NaCl + 1mM EDTA
50mM Phosphate Buffer pH5.8 (Fisher S-374,792035 NaPhos.Dibasic)
50mM Tris-HCL pH 8.0
50mM Tris-HCL + 20mM EDTA pH 8.0
50mM Tris-HCL 8.5 + 5mM NaCl
Diatom Suspension
Diatom Isolation Reagents
Sterile TE
PCR mix
Taq Polymerase
SK4.3 Plasmid
Primer Sequence I.D. No: 5 and 6
$\beta$-actin + control
$\beta$-actin primer Sequence I.D. No: 3 and 4
Parafin Oil (J.T. Baker, Phillipsburg, NJ) 10% acrylamide gel
10 X TBE
50 X TAE
1% agarose in TAE
Ethidium Bromide
MWM (molecular weight markers) for 10% acrylamide gel (HINFI restriction digest of bacteriophage $\phi$X174 DNA)
MWM for 1% agarose gel (HIND III restriction digest of lambda bacteriophage DNA (BRL Cat. no. 5612SA): 23.1, 9.4, 6.6, 4.4, 2.3, 2.0 kilobases)
Milli Q $H_2O$ (Millipore Corp. Bedford, MA)
loading dye (25% Ficoll 400, 25% Bromophenol Blue, 25% Xylene cyanol)
Polaroid 57 film (Polaroid, Cambridge, MA)
Polaroid 55 film

Procedure:

Fluid from clinical sample was set aside. Each swab was washed by vortexing in .5ml aliquots of sterile $H_2O$ until total volume of about 4.5 ml. 45 $\mu$l of SK4.3 plasmid was added.
Sample was divided into 9, .5ml, samples and centrifuged to pellets. Supernatant was discarded.
One tube pellet was resuspended in 150$\mu$l sterile TE and set aside for PCR untreated control.
One of each of the remaining 8 pellets was treated by one of the following enzyme protocols, resuspending the pellet in 500 $\mu$l of the respective buffer.

```
                                                            UNITS

#1  Achromopeptidase – 36μl of 5mg/ml                        100

     Buffer: in 100 mM Trisma 9.0 + 10mM NaCl + 1mM EDTA Buffer



#2  Mutanolysin – 50μl of 1mg/ml                             250

     Buffer:  50 mM Phosphate Bufer pH 5.8
```

| SAMPLE | QUANTITY | STERILE $H_2O$ |
|---|---|---|
| Untreated | 10μl | 40μl |
| Achromopeptidase | 10μl | 40μl |
| Mutanolysin | 10μl | 40μl |
| Proteinase K | 10μl | 40μl |
| Proteinase K, EDTA | 10μl | 40μl |
| Trypsin | 10μl | 40μl |
| Lyticase | 10μl | 40μl |
| Lysing Enzymes A | 10μl | 40μl |
| Lysing Enzymes B | 10μl | 40μl |
| $\beta$-actin + Control | 1μl | 49μl |
| SK4.3 + Control | 10μl | 40μl |
| -Control (sterile $H_2O$) | - | 50μl |

The following PCR mix was made up:    650μl PCR buffer
6.5μl Taq Polymerase (Amplitaq Polymerase, Perkin-Elmer Cetus, Norwalk,CT) 13μl each Sequence I.D. No:
3 and 4
13μl each SK4.3 Sequence
I.D. 5 and 6

50μl of the mix was added to each tube and overlayed with parafin oil.

Incubation for PCR was as described for PCR protocol.

10μl of each sample and control was mixed with 2 μl loading dye and run on a 10% acrylamide gel at 100 v for about 45 minutes. Running buffer was 1 X TBE. Gel was stained for 15 minutes with dilute Ethidium Bromide in Milli Q $H_2O$, backstained in Milli Q $H_2O$ only for 15 minutes and photographed 1 second with 57 film and 10 second 55 film. Negative of 55 film was developed and read by densitometry in a Gelman ACD densitometer.

20μl of each sample was combined with 2μl loading dye and run on a 1% agarose gel for about 30 minutes at 100 v. Gel was run as horizontal submarine gel with 1 X TAE running buffer. Gel was stained, destained and photographed with 57 film 1 second as above.

Enzyme Study for Beta-actin and SK4.3 (Gelman ACD (relative densitometric units)

|  | Beta-Actin | SK4.3 |
|---|---|---|
| Achromopeptidase | 0.00114 | 0.00738 |
| Mutanolysin | 0.00701 | 0.00547 |
| Proteinase K | 0.00219 | 0.00599 |
| Proteinase K, EDTA | 0.00511 | 0.00637 |
| Trypsin | 0.00459 | 0.00642 |
| Lyticase | 0.00314 | 0.00728 |
| Enzymes A | 0.00407 | 0.00552 |
| Enzymes B | 0.00696 | 0.00538 |

All the enzymes aided sample processing. Achromopeptidase and Lyticase treatment are the best for amplification of SK4.3. Mutanolysin and Enzymes B are best for $\beta$-actin. All protocols worked reasonably well for SK4.3 plasmid.

EXAMPLE 2

Purpose:

To determine if sample processing using N-acetyl-L-cysteine in NaOH/NaCitrate buffer will aid in target and co-target amplification. Sample processing will be used with and without Proteinase K, EDTA (Sigma, St. Louis, MO) treatment.

Materials:

Two clinical samples (cervical swabs)
SK4.3 Plasmid
SK4.3 Primer Sequence I.D. No: 5 and 6
$\beta$-actin + control
$\beta$-actin Primer Sequence I.D. No: 3 and 4
Taq Polymerase
PCR buffer
Proteinase K
EDTA
Sterile $H_2O$
Sterile TE
Diatom Suspension
Diatom Isolation Reagents
N-acetyl-L-cysteine (Sigma #A-7250 Lot #7F-0397 NaOH (Fisher Cat. #SS266-1 Lot #885110-24)
NaCitrate (Fisher Scientific Cat #S-279 Lot #851197)
Phosphate Buffer (50 mM $NaH_2PO_4$/$NaHPO_4$, pH 7.0)
Parafin Oil
Loading Dye
10% acrylamide gel
10 X TBE
MWM
57 Polaroid film
55 Polaroid film
Ethidium Bromide
Milli Q $H_2O$

The clinical samples each received 7.5$\mu$l of SK4.3 plasmid, then each was divided into 3 aliquots of 0.5ml volume. Each aliquot received a separate treatment:

either 1) addition of 0.5% Nalc* (0.5 ml) (1 aliquot "a")
or 2) addition of 1.0% Nalc* (0.5 ml)(2 aliquots"b","c")

and was then incubated at room temperature for 30 minutes. The samples were then neutralized by addition of phosphate buffer and centrifuged for 15 minutes (2500 X g). The pellets were kept and the supernatants discarded. Each pellet was resuspended in 0.5 ml sterile $H_2O$; one aliquot ("c") received $50\mu l$ each of proteinase K and EDTA solutions. After incubation at 55° for 30 minutes the reaction mixtures were centrifuged for 2 minutes in a microcentrifuge and the supernatants were discarded. The pellets were then further purified on diatoms, and purified DNA was eluted in $150\mu l$ TE buffer.

* 0.5% Nalc = 0.05 gram of N-acetyl-L-cysteine in 10ml 0.1M sodium citrate/0.5N NaOH

1.0% Nalc = 0.1 gram of N-acetyl-L-cysteine in 10ml 0.1M sodium citrate/0.5 N NaOH

The following samples were made up for PCR:

| TUBE | SAMPLE | AMOUNT | STERILE $H_2O$ |
|------|--------|--------|----------------|
| 2 | 1a (.5%)(with SK4.3) | $10\mu l$ | $40\mu l$ |
| 3 | 1b (1%)(with SK4.3) | $10\mu l$ | $40\mu l$ |
| 4 | 1c (1% Pro K/EDTA) (with SK4.3) | $10\mu l$ | $40\mu l$ |
| 5 | 2a (5% with SK4.3) | $10\mu l$ | $40\mu l$ |
| 6 | 2b (1% with SK4.3) | $10\mu l$ | $40\mu l$ |
| 7 | 2c (1% with Pro K, EDTA, SK4.3) | $10\mu l$ | $40\mu l$ |
| 8 | + Control ($\beta$-actin/SK4.3) $1\mu l/5\mu l$ | $10\mu l$ | $40\mu l$ |
| 9 | -Control - Sterile $H_2O$ | -- | $50\mu l$ |

To each sample was added $50\mu l$ of the following PCR mix:

$400\mu l$ PCR buffer

$4\mu l$ Taq Polymerase

$8\mu l$ each $\beta$-actin Primer Sequence I.D. No: 3 and 4

$8\mu l$ each SK4.3 Primer Sequence I.D. No: 5 and 6

The tubes were overlayed with $50\mu l$ Parafin oil and subjected to PCR amplification.

$10\mu l$ of each sample and control was mixed with $2\mu l$ loading dye and run on a 10% acrylamide gel for about 45 minutes at 100 v. 1 X TBE was used as running buffer.

Gel was stained 15 minutes with dilute Ethidium Bromide in Milli Q $H_2O$, back-stained with Milli Q $H_2O$ only for 15 minutes, then photographed with Polaroid 57 film for 1 second under uv light.

Gel was also photographed with Polaroid 55 film under uv light for 10 seconds. Negative was developed for possible future densitometry readings.

Results

The appropriate sized amplified target sequence was observed after each treatment, indicating that the Nalc/NaOH treatment alone or with subsequent Proteinase K/EDTA treatment did not adversely affect the target DNA or its amplification by PCR.

Conclusion

The Nalc/NaOH treatment, as well as proteinase K/EDTA digestion, is compatible with the PCR amplification.

EXAMPLE 3

Purpose:

To screen a series of enzymes for lysis efficiency of M. tuberculosis.

Materials:

Lysozyme (Sigma Cat #L6876 Lot #39F-8210)

Achromopeptidase (Sigma Cat #A-3547 Lot #88F-0799)

Lipoxidase (Sigma Cat #L-7395 Lot #118F-03421)

Mixed Glycosidase A (from C. lamdas Sekagaku Lot #FL 84803)

Zymolase 20T (Sekagaku/Miles Cat #32092-1 Lot #2)

Mixed Glycosidase B (from T. cornutus Seikagu Lot #ET 84701)

M. tuberculosis - cultured and heat inactivated Lysing Enzymes A (from Tricoderma Larzanium Sigma Cat #L2265 Lot #36F-0807)
Lysing Enzymes B (from Rhizoctonia Solani Sigma Cat #L8757 Lot #46F-0273)
Phospholipase A (Sigma Cat # D-6534 Lot #1298-8005)
Lipase (Sigma Cat # M-9901 Lot #88F-02081)
Mutanolysin (Sigma Cat # M-9901 Lot #98F-68211)
Achromopeptidase/Mutanolysin cocktail (10 mg/1mg)
GEN-PROBE Lysing Reagent Tube (Lot #92084)
1:1 phenol:chloroform (Tris saturated BRC 55040A Lot #71209/Baxter B&J Cat #67-66-3 Lot #A W342)
Ethanol (Fisher E-575-500 Lot #897309) 70%
3M Sodium Acetate (pH 5.5)
Lyophillizer (Savant Speed Vacuum)
TE buffer

Procedure:

1. Into 13 tubes was placed 2 ml of pelleted M. tuberculosis (after decanting).
2. To each portion was added 90µl $H_2O$ (except the gen probe tube, which got 100µl) and 10µl of the enzyme so that final concentration was 500 µg/ml (50 µg total).
3. All tubes were incubated at 37°C for 30 minutes (gen probe run according to protocol).
4. 200µl of water was added to each tube to bring volume up to 300µl, then 2 phenol:chloroform extractions were performed.
5. 30µl of 3M sodium acetate and 600µl ethanol was added to the aqueous layer, and this mixture incubated at -20°/C for about 2 1/2 days.
6. Samples were spun at about 4°C for twenty minutes, excess ethanol was removed, and pellets were dried in the lyophillizer for 1 hour.
7. Samples were resuspended in 20µl TE and 3µl loading buffer, and electrophoresed 35 minutes at 150v (94 vh) (1% agarose gel in 1 X TA buffer).
8. Gel was ethidium bromide stained (1µg/1ml) and visualized under uv light.
The results show that achromopeptidase results in a distinct band at 23 KB, corresponding to genomic DNA. The achromopeptidase, which also has mutanolysin, shows less of a distinct band.

EXAMPLE 4

Explanation of experiments:

Slot blot experiments confirm results obtained by PCR which indicate that achromopeptidase was the most efficient enzyme for use in M. tuberculosis lysis.

Materials and Methods:

Probe = 81 mer HINFI/SCRFI fragment Nucleotides 140-220 of IS6110
Samples as for Example 3
* Samples were treated as in previous experiment.
10µl DNA was denatured in 10 µl 0.5 N NaOH for 10 minutes, room temperature, to which was added 50 µl (.125 X SSC, .125 NaOH) for MTB samples or 180 µl for SK 4.3 (22.5 ng/µl X 10 µl) (=225 ng total).
The samples were applied to Gene Screen (Dupont), then suction applied.
Each slot was washed in 6 X SSC., then air dried and treated as suggested by the manufacturer.

OBSERVATIONS

i) Achromopeptidase gave the greatest hybridization signal in a variety of different protocols and replicates.
ii) Proteinase K gave a strong response, but less than achromopeptidase.
iii) Mutanolysin gave a weak hybridization.
iv) No (or very little) response was seen with trypsin treatment or with achromopeptidase and guanidine.

## Discussion

The data suggests that the best treatments are enzymatic: achromopeptidase, lyticase and proteinase K. These were followed by the method of Brisson-Noël et al., The Lancet Nov **4**:1069 (1989), disinfectants (Chlorox, Staphene and Lysol), SDS and Tween 20. A higher concentration of Chlorox (10% versus 1%) resulted in loss of signal, suggesting that the higher concentration denatures or destroys the DNA. The disinfectants liberated hybridizable DNA and can be used alone or after another treatment (e.g. the enzymatic treatments mentioned above) in order to disinfect the sample without destroying the DNA.

## Conclusions

The sample lysis/disinfection methods released intact DNA from the M. tuberculosis organisms.

## EXAMPLE 5

### Purpose:

To determine the best enzyme treatment and conditions for releasing and amplifying M. tuberculosis DNA in spiked clinical samples already NaLc treated.

### Materials:

Clinical Samples: Sputum #3961, #3979 untreated; smear negative, culture confirmation is pending.
BACTEC - cultured M. tuberculosis
Enzymes, buffers and materials
PCR mix, 10 X PCR buffer, 20 mM $\beta$ME ($\beta$-mercaptoethanol) 4.4 mM $MgCl_2$, 5mMdNTPS
Sterile TE (10mM Tris-HCL, 1mM EDTA)
Proteinase K
IS6110 + Control
$\beta$ME ($\beta$-mercaptoethanol) - for PCR mix (Sigma Lot #48F-07011, Order #M6250)

### Procedure:

Sputum samples were combined to about 8 ml.
14 mls of Mtb suspension ($10^6$/mL was aliquoted in 1 ml amounts and pelleted, supernatant discarded.
To each tube was added .5 ml sputum and tubes were vortexed 30 seconds. The samples were divided into 2, 7 tube sets. To one set was added .5 ml to each tube of the following solution: 1% NalC + vortexed briefly.
1 gm N-acetyl-L-cysteine
10 ml NaOH/NaCi solution
The untreated set was put aside while the NaIC treated set incubated at room temperature for 20 minutes. 4 mls of phosphate buffer was added to neutralize samples.
4 mls of phosphate buffer were added to neutralize each sample, then they were centrifuged for 15 minutes at highest speed on a clinical centrifuge. Supernatants were discarded and the pellets were treated as follows:

| Pellet | Enzyme | Buffer |
|---|---|---|
| 1 | achromo* | TE |
| 2 | Pro K" / EDTA | TE |
| 3 | achromo / pro K / EDTA | TE |
| 4 | achromo / pro K / EDTA | 8 M guanidine |
| 5 | mutano + / achromo | TE |
| 6 | achromo / pro K / EDTA / mutano | TE |
| 7 | control - untreated | TE |

(*achromopeptidase, "proteinase K, + mutanolysin)

The reactions were incubated at $55^0$C for 1 hour, then microfuged for 5 minutes and the supernatants discarded. All 14 samples (enzyme treated and respective controls) were purified by adsorption to diatoms and eluted in 100 $\mu$l TE buffer.

10$\mu$l of each sample was mixed with 90$\mu$l sterile TE buffer, then 10$\mu$l of that dilution was mixed with 40$\mu$l TE for the PCR reaction. 50$\mu$l of the following PCR mix was added to each sample:

900$\mu$l PCR buffer mix

9$\mu$l Taq polymerase

18$\mu$l each $\beta$-actin Sequence I.D. No: 3 and 4

18$\mu$l IS6110 primer sequence I.D. No: 5

9$\mu$l IS6110 primer sequence I.D. No: 6

Each reaction was overlaid with 50$\mu$l parafin oil and subjected to 30 cycles of PCR at the following temperatures: $94^0$C, 1 minute; $62^0$C, 1 minute; $72^0$C, 1 minute.

10$\mu$l of each PCR reaction was run on a 10% acrylamide gel for about 40 minutes, stained, backstained, and photographed with Polaroid 57 film under uv light for 1 second.

Results

All of the tested conditions (except negative controls) produced amplification of both $\beta$actin and IS6110 DNA with $10^4$ targets added. However, after Nalc treatment there appeared to be better amplification, as evidenced by brighter flourescence of the ethidium-stained target bands and this enhancement was best seen for the (upper) actin target. In the samples that were not treated with Nalc, achromopeptidase treatment gave the best amplification of both $\beta$actin and IS6110 targets. After Nalc treatment, however, all of the enzyme treatments gave the same high level of amplification for both targets.

Conclusion

In the presence of Nalc treatment the various enzyme treatments did not inhibit the amplification of $10^4$ human $\beta$actin or Mtb IS6110 target sequences.

EXAMPLE 6

Purpose:

To determine whether sample liquification, M. tuberculosis lysis and disinfection can be combined into the same protocol.

Materials:

7 clinical sample aliquots

Proteinase K

EDTA

Mutanolysin

Achromopeptidase

Diatom Suspension (Sigma, St. Louis, MO)

Diatom Isolation Reagents

N-acetyl-L-cysteine (Sigma, St. Louis, MO)

NaOH, NaCitrate Buffer

Phosphate Buffer

MTB culture

Sterile $H_2O$

Sterile TE

PCR buffer mix

Primers $\beta$-actin Sequence I.D. No: 3 and 4

Primers MTB (SK4.3) (Seq ID No: 5 and 6)

+Control $\beta$-actin

+Control MTB (SK4.3)

Taq Polymerase

Parafin Oil

10% acrylamide gel
10 X TBE
Loading Dye

Following:

36$\mu$l Achromopeptidase
50$\mu$l Proteinese K
50$\mu$l EDTA
50$\mu$l Mutanolysin

Samples + diatom control were incubated at 55° C for 1 hour, centrifuged 3 minutes, and supernatant discarded. Pellets were processed by diatom purification/isolation protocol. Samples were eluted in about 100$\mu$l TE (75 + 50 $\mu$l) (to account for pellet absorption).

10$\mu$l of each sample and both controls were mixed with 90 $\mu$l sterile $H_2O$, 10 $\mu$l of that mixture combined with 40 $\mu$l sterile $H_2O$ for PCR. Positive and negative controls were added for PCR. 50$\mu$l of PCR mix was added to each tube. Each tube was overlayed with 50$\mu$l parafin oil and subjected to PCR.

10$\mu$l of each sample (including the controls) were run on a 10% acrylamide gel for about 45 minutes, stained, backstained and photographed under uv light. Running buffer was 1 X TBE.

Results:

Multiple additional bands were observed along with the target amplification throughout all our samples and controls, indicative of inaccurate priming.

Negative control did not show amplification of the specific DNA targets though multiple smaller nonspecific DNA targets were amplified.

Similar nonspecific amplification banding was seen throughout all samples. The positive control did amplify specifically.

The only samples showing $\beta$-actin amplification under these conditions were Hibiclens 1%, and Roccal II.

EXAMPLE 7

Purpose:

To repeat the PCR portion of previous experiment using heat-inactivated M. tuberculosis to spike clinical sputum samples, and to determine the minimum number of organisms detectable by PCR after sample processing, lysis, and diatom recovery.

Materials:

Eluted samples from previous experiment
PCR mix
Taq Polymerase
Primer Sequence I.D. No: 3 and 4
$\beta$-actin + control
Sterile TE
Parafin oil
Loading dye
10% acrylamide gels
Ethidium Bromide
MWM
Milli Q $H_2O$
Polaroid 57 film
10 X TBE
IS6110 primer Sequence I.D. No: 5 and 6
Five pooled clinical sputum samples, smear (-), not culture confirmed. Total volume about 50 ml.

12

Procedure:

1.1 ml M. tuberculosis suspension was removed aseptically from culture bottle and placed in a 1.5 ml Eppendorf tube. 100 μl was transferred to a 900 μl sterile TE blank. This procedure was repeated until final dilution was 10 organisms/ml. Total tubes = 6.

All samples were centrifuged under hood 5 minutes on high, supernatants removed by syringe and discarded in disinfectant. Pellets were each resuspended in 100 μl sterile TE.

Sputum sample was evenly divided into 7 (50 ml) centrifuge tubes (approximately 7.5 ml).

To one of six tubes was added one of the 100 μl dilutions of M. tuberculosis and spiked sample was vortexed 30 seconds.

To all samples was added an equal volume of 1% NaLc solution. Samples were mixed by inverting several times and incubated at room temperature for 20 minutes. About 4 volumes of 50 mM phosphate buffer were added (total volume = 50ml) and the samples were centrifuged at highest speed in a clinical centrifuge for 15 minutes.

The supernatants were discarded and the pellets were resuspended in 1 ml O-SYL disinfectant solution. After 10 minutes incubation at room temperature, the samples were transfered to 1.5 ml tubes and centrifuged in an eppendorf centrifuge for 3 minutes. Supernatants were discarded and the pellets were resuspended in 0.5 ml sterile TE buffer.

Each tube received:

18 μl 10mg/ml achromopeptidase

50 μl 1 mg/ml Proteinase K

50 μl 100 mM EDTA.

The reactions were incubated at $55^0$C for 1 hour. DNA was isolated by adsorption to diatoms and eluted in 100 μl TE buffer. 10 μl of each DNA sample was combined with 40 μl sterile TE buffer for PCR. To each reaction was added 50 μl of the following PCR buffer:

475μl PCR buffer mix

5μl Taq Polymerase

10μl each β-actin Sequence I.D. NI: 3 and 4 primers

10μl IS6110 primer Sequence I.D. No: 5 (25 mM)

5μl IS6110 primer Sequence I.D. No: 6 (50 mM)

Each tube was overlayed with 50μl Parafin oil and subjected to temperature cycling as follows:

$94^0$C, 1 minute; $62^0$C, 1 minute; $72^0$C 1 minute. This was repeated for 30 cycles, followed by extension at $72^0$C for 7 minutes and finally held at $4^0$ until analysis.

10μl of each was mixed with 2μl loading dye and run on a 10% acrylamide gel at 100 v for about 45 minutes. Gel was stained 15 minutes, backstained 15 minutes and photographed under uv light.

Results: Ethidium-stained gel profile of the products of the PCR reaction:

| Total targets/reaction | | Actual target dilution | Visual score |
|---|---|---|---|
| 1 | MWM | | |
| 2 | $10^6$ | $(10^5)$ | + + |
| 3 | $10^5$ | $(10^4)$ | + |
| 4 | $10^4$ | $(10^3)$ | + |
| 5 | $10^3$ | $(10^2)$ | + |
| 6 | $10^2$ | (10) | + |
| 7 | 10 | (1) | + |
| 8 | 0 | 0 | -- |
| 9 | + control | | + + + |
| 10 | -control | | -- |

(+) = weakly fluorescent amplified target observed

(+ +) = strong fluorescent amplified target observed

(+ + +) = very intense fluorescent amplified target observed (--) = no amplified target observed

13

Negative control was negative for amplification. Positive control was positive for specific amplification of DNA of expected size for IS6110. The method allowed detection of approximately 1 to 10 M. tuberculosis organisms.

TABLE 1

SUMMARY OF SAMPLE PROCESSING AND LYSIS PROTOCOLS TESTED

| PROTOCOLS | DNA | Beta-Actin | | IS6110 | |
|---|---|---|---|---|---|
| | | w/ | w/o | w/ | w/o |
| **Basics** | | | | | |
| SDS (Life Technologies Inc, Gaithersburg, MD) | nd | nd | nd | nd | nd |
| DTT (Calbiochem, San Diego, CA) | nd | nd | nd | nd | nd |
| SDS/DTT | nd | -/+ | -/+ | nd | nd |
| NaOH(.5N) | | + | nd | nd | nd |
| Sucrose Lysis | nd | -/+ | -/+ | nd | nd |
| Proteinase K | ++ | ++ | nd | nd | ++ |
| EDTA | ++ | ++ | nd | nd | ++ |
| Pro.K/EDTA | ++ | ++ | nd | ++ | nd |
| Boil/Pro.K/EDTA | +++ | ++ | nd | ++ | nd |
| Boil/Pro.K | ++ | ++ | nd | ++ | nd |
| Boil/EDTA | ++ | ++ | nd | ++ | nd |
| Boil | + | ++ | + | nd | ++ |
| Chelex/Boil (Biorad, Richmond,CA) | nd | - | -/+ | nd | nd |
| **Purification Protocols** | | | | | |
| Prep-A-Gene | ++ | ++ | + | + | nd |
| Isoquick (Microprobe Corp, Bothell, WA) | -/+ | ++ | -/+ | + | nd |
| Diatoms | + | ++ | + | ++ | nd |
| Panasol (Danbaxy Labs, Inc. Sterling, VA) | - | -/+ | -/+ | ++ | nd |
| Strataclean (Stragene, LaJolla,Ca) | - | - | -/+ | + | nd |
| EtOH | nd | ++ | + | nd | nd |
| **Enzymes** | | | | | |
| Achromopeptidase | -/+ | nd | -/+ | nd | +++ |
| Mutanolysin (Mut) | + | nd | +++ | nd | + |
| Pro.K | -/+ | nd | + | nd | + |
| Pro.K/EDTA | -/+ | nd | ++ | nd | ++ |
| Trypsin | -/+ | nd | ++ | nd | ++ |
| Lyticase | + | nd | + | nd | +++ |
| Sigma Lysing A | -/+ | nd | + | nd | + |
| Sigma Lysing B | + | nd | +++ | nd | + |

Combinations
(with NaLc)

| | | | | | |
|---|---|---|---|---|---|
| Achr/Pro.K/EDTA(Tris) | nd | nd | -/+ | nd | +++ |
| Ach/Pro.K (8M Guan) | nd | nd | -/+ | nd | ++ |
| Mut/Achromo | nd | nd | -/+ | nd | ++ |
| Mut/Ach/Pro.K/EDTA | nd | nd | -/+ | nd | +++ |

NaLc Treatment

| | | | | | |
|---|---|---|---|---|---|
| 05% | nd | nd | ++ | nd | + |
| 1.0% | nd | nd | ++ | nd | ++ |
| 1.0% w/Pro.K/EDTA | nd | ++ | nd | +++ | nd |

Disinfectants

| | | | | | |
|---|---|---|---|---|---|
| Bleach 1% | - | nd | -/+ | nd | -/+ |
| Bleach 5% | - | nd | -/+ | nd | -/+ |
| Amphyl 1% (National Labs, Montvale, NJ) | -/+ | nd | + | nd | + |
| Amphyl 5% | -/+ | nd | + | nd | + |
| Lysol 1% National Labs, Montvale, NJ) | -/+ | nd | + | nd | + |
| Lysol 5% | -/+ | nd | + | nd | + |
| SDS 1% | - | nd | -/+ | nd | -/+ |
| SDS 5% | - | nd | -/+ | nd | -/+ |
| Hibiclens 1% (Stuart Pharmaceuticals, Wilmington, DE) | + | nd | ++ | nd | +++ |
| Hibiclens 5% (Stuart Pharmaceuticals, Wilmington, DE) | + | nd | ++ | nd | + |
| Tween 80 1% | + | nd | +++ | nd | +++ |
| Tween 80 5% | + | nd | ++ | nd | + |
| Vesphene (1:128)* (Vestal) | + | nd | ++ | nd | + |
| Roccal II(1:64)* (National Labs, Montvale, NJ) | ++ | nd | ++ | nd | + |
| O-SYL (1:128)* (National Labs, Montvale, NJ) | + | nd | +++ | nd | ++ |
| Staphene (1:128)* Calgon, St. Louis, MO) | -/+ | nd | ++ | nd | -/+ |
| Glutarex (1:23)* (3M, St Paul,MN) | - | nd | + | nd | -/+ |
| Cidex-Plus (1:125)* (Surgikos, Arlington, TX) | - | nd | + | nd | -/+ |

\* = Made up as recommended by manufacturer.

| Endonucleases | | | | | |
|---|---|---|---|---|---|
| Hind III | nd | + | + | + | + |
| EcoRI | nd | + | + | + | + |
| BamHI | nd | + | + | -/+ | -/+ |
| DdeI | nd | + | + | -/+ | -/+ |
| XhoI | nd | + | + | -/+ | -/+ |
| HincII | nd | + | + | -/+ | -/+ |

| Primers | | | | | |
|---|---|---|---|---|---|
| Clontech (b-A) 1+2 | nd | +* | +*(*EtOH Precip) | nd | nd |
| Seq I.D. No: 1 & 2 (b-A) | nd | + | + | nd | nd |
| Seq I.D. No: 3 & 4 (b-A) | nd | ++ | + | nd | nd |
| Seq I.D. No: 1 & 44 (b-A) | nd | nd | – | nd | nd |
| IS6110 (Mtb) Seq I.D. No. 5 & 6 (74 mer) | nd | nd | nd | ++ | + |
| IS6110 (Mtb) Seq ID No: 4 & 7 | nd | nd | nd | + | nd |

| Organic Solvents | | | | | |
|---|---|---|---|---|---|
| DMSO 2% | nd | nd | + | nd | nd |
| DMSO 5% | nd | nd | ++ | nd | nd |
| DMSO 10% | nd | nd | + | nd | nd |
| DMSO 15% | nd | nd | + | nd | nd |
| Glycerol 2% | nd | nd | + | nd | nd |
| Glycerol 5% | nd | nd | + | nd | nd |
| Glycerol 10% | nd | nd | + | nd | nd |
| Glycerol 15% | nd | nd | + | nd | nd |
| PEG 2% | nd | nd | + | nd | nd |
| PEG 5% | nd | nd | + | nd | nd |
| PEG 10% | nd | nd | + | nd | nd |
| PEG 15% | nd | nd | + | nd | nd |

Legend: –, no result; -/+, low result; +, positive or equal to control; ++, strong positive; +++, very strong positive; nd, not done/not applicable; w/, with Pro.K/EDTA treatment; w/o, without Pro.K/EDTA treatment.

TABLE II

| Method | Comments | DNA | PCR | Blot |
|---|---|---|---|---|
| Published Methods: | | | | |
| M.Roberts et al *1 | NaCl/ethanol | − | nd | nd |
| P.Chomczynski et al *2 | GuSCN/Phenol | − | − | − |
| A.Brisson-Noel et al*3 | NaOH,95°/NaCl/SDS | + | ++ | ++ |
| U.Sjobring et al *4 | ProK/SDS,55° | + | − | − |
| D.De Wit et al*5 | Tris/Salt/EDTA,70° | + | ++ | + |
| D.Whipple et al*6 | Enzymes/freeze thaw | + | − | nd |
| R.Boom et al *7 | Celite/GuSCN | ++ | + | + |
| | | | | |
| Chaotropes/Organic solvents: | | | | |
| DMSO | DMSO/NaOH/SDS | + | nd | nd |
| Acetone | Acetone/NaOH/SDS | − | nd | nd |
| GuHCl | Celite/GuHCl | -+ | ++ | − |
| NaClO$_4$ | Celite/NaClO$_4$ | + | nd | nd |
| Phenol/chlorform | | − | + | − |
| NALC/NaOH | N-acetyl CYS/NaOH/ Citrate | − | − | + |
| | | | | |
| Mechanical: | | | | |
| Microwave | | − | nd | nd |
| Boiling | | − | + | + |
| Sonication | | − | − | − |
| exothermic reaction | Thiodiethanol/Urea/ H2O2 | − | − | nd |
| Boiling & beads | | − | + | − |
| Sonication & beads | | − | + | − |
| Gen-Probe method (Gen Probe, San Diego, CA) | Boiling/sonication/ beads/SDS | nd | + | + |
| | | | | |
| Detergents: | | | | |
| SDS | 1%,100° | nd | − | ++ |
| Tween 80 | 1%,20° | + | + | + |
| | | | | |
| Enzymes: | | | | |
| Achromopeptidase | 100 units,50°,30 min | + | ++ | ++ |
| Lipoxidase | 50 ug, 37°, 30 min | + | nd | nd |
| Glycosidase B | 50 ug, 37°, 30 min | + | nd | nd |
| Lysing enzymes A | 50 ug, 37°, 30 min | − | + | − |
| Lysing enzymes B | 50 ug, 37°, 30 min | + | − | − |
| Mutanolysin | 250 units,37°,120 min | − | + | + |
| Trypsin | 1000 units,37°,120 min | − | − | − |
| Thermolysin | 100 ug, 70°, 120 min | − | + | − |
| Lyticase | 285 units,37°,120 min | + | + | ++ |
| Proteinase K | 100 ug, 50°, 120 min | + | + | ++ |
| Pepsin | 100 ug, 37°, 120 min | − | + | − |
| Papain | 100 ug, 37°, 120 min | − | − | − |

```
Pronase          100 ug, 37°, 120 min      +    +    −
Chymotrypsin     100 ug, 37°, 120 min      −    −    −
Chymopapain      100 ug, 37°, 120 min      −    −    −
V8 protease      100 ug, 37°, 120 min      −    +    −

Disinfectants:
Chlorox          1%                        +    +    +
Chlorox          10%                       +    +    −
Lysol            10%                       +    +    +
(National Labs,
 Montvale, NJ)
Staphene         5%                        +    +    +
(Calgon, St. Louis,
 MO)
```

Legend:−, no result; +, result; ++, strong result; nd, not determined
"DNA": Amount of DNA determined by OD260/280 or estimated on EtBr−stained gel. "PCR": Amount of PCR product observed on EtBr−stained gel. "Blot: Amount of labeled probe hybridized to the M.tb DNA on slot blot.

[1]    J. of Clin. Microbiology 25:1239 (1987)
[2]    Analytical Biochemistry 162:156 (1987)
[3]    The Lancet Nov 4:1069 (1989)
[4]    J. of Clin. Microbiology 28:2200 (1990)
[5]    J. of Clin. Microbiology 28:2437 (1990)
[6]    J. of Clin. Microbiology 25:1511 (1987)
[7]    J. of Clin. Microbiology 28:495 (1990)

## SUMMARY OF SAMPLE PROCESSING DATA

The intention of the "basics" was to demonstrate the ability to amplify the Mycobacterium sequence IS6110 or human cytoplasmic beta act in after a variety of basic sample treatments. The human actin target was used as a control to show which treatments were of general use and not just specific for use with IS6110. It can be seen that Proteinase K or EDTA treatments do not inhibit the amplification of either the human beta actin or Mycobacterium targets. Therefore, these treatments are compatible with the sample processing method and it was confirmed that utilizing both treatments simultaneously worked, as evidenced by the amplification of both the actin and mycobacterium DNA targets after proteinase K/EDTA treatment. Note that this treatment does not use denaturating conditions such as heating to 95°C or boiling and therefore produces double stranded DNA. Likewise, the denaturating conditions which were tested (i.e., boiling alone or boiling with proteinase K or EDTA) were compatible with amplification of both the human actin target and the Mycobacterium targets (would produce single-stranded DNA).

A variety of purification protocols were tested to determine which was the best for obtaining a DNA target from a reaction mixture in a way that was most amenable to DNA amplification. The best conditions for IS6110 were the solid phase adsorption method on diatoms (trade name: CELITE, Celite Corp., Denver, Colorado) or PANASOL, a liquid extraction reagent from Panbaxy (Sterling, Va). Since the liquid extraction did not produce good amplification of the human actin target, diatom capture was determined to be the best general method for the DNA purification.

Next, the enzymes used for lysis of the Mycobacteria were tested for their effects on PCR amplification of the human actin and IS6110 targets. Of the enzymes tested, achromopeptidase and lyticase produced the best amplification of the IS6110 target while mutanolysin and the commercially prepared enzyme mixture, Sigma Lysing B (Sigma, St. Louis, Mo) were best for the human beta actin target. Therefore proteinase K, which was compatible with actin amplification and achromopeptidase, which was compatible with IS6110 amplification and effective for lysis of Mycobacteria were combined in the protocol.

18

Finally, when the optimal treatments for the various steps (i.e., sample disruption, mycobacterial lysis, DNA capture) were combined in the presence of sputum liquification reagents ("NALC"), they were found to be compatible with amplification of the IS6110 target.

Although the invention has been described with respect to specific modifications, the details thereof are not to be construed as limitations, for it will be apparent that various equivalents, changes and modifications may be resorted to without departing from the spirit and scope thereof, and it is understood that such equivalent embodiments are to be included therein.

```
                              SEQUENCE LISTING


    (1) GENERAL INFORMATION:

         (i) APPLICANT: Down, James A
                        Keating, William E
                        Dey, Margaret S
                        Siddiqi, Salman U

        (ii) TITLE OF INVENTION: Release of Intracellular Components

       (iii) NUMBER OF SEQUENCES: 6

        (iv) CORRESPONDENCE ADDRESS:
             (A) ADDRESSEE: Richard J. Rodrick
             (B) STREET: 1 Becton Drive
             (C) CITY: Franklin Lakes
             (D) STATE: New Jersey
             (E) COUNTRY: U.S.A.
             (F) ZIP: 07417-1880

         (v) COMPUTER READABLE FORM:
             (A) MEDIUM TYPE: Floppy disk
             (B) COMPUTER: IBM PC compatible
             (C) OPERATING SYSTEM: PC-DOS/MS-DOS
             (D) SOFTWARE: PatentIn Release #1.0, Version #1.25

        (vi) CURRENT APPLICATION DATA:
             (A) APPLICATION NUMBER:
             (B) FILING DATE:
             (C) CLASSIFICATION:

      (viii) ATTORNEY/AGENT INFORMATION:
             (A) NAME: Stierwalt, Brian K
             (B) REGISTRATION NUMBER: 33,213
             (C) REFERENCE/DOCKET NUMBER: P-2262

        (ix) TELECOMMUNICATION INFORMATION:
             (A) TELEPHONE: 201-847-5317
             (B) TELEFAX: 201-848-9228


    (2) INFORMATION FOR SEQ ID NO:1:

         (i) SEQUENCE CHARACTERISTICS:
             (A) LENGTH: 24 base pairs
             (B) TYPE: nucleic acid
             (C) STRANDEDNESS: single
             (D) TOPOLOGY: linear
```

        (xi) SEQUENCE DESCRIPTION: SEQ ID NO:1:

TAAGAACTCG GCGCGCCGGA AGTG                                        24

    (2) INFORMATION FOR SEQ ID NO:2:

        (i) SEQUENCE CHARACTERISTICS:
            (A) LENGTH: 24 base pairs
            (B) TYPE: nucleic acid
            (C) STRANDEDNESS: single
            (D) TOPOLOGY: linear


        (xi) SEQUENCE DESCRIPTION: SEQ ID NO:2:

GACGACGAGC GCGGCGATAT CATC                                        24

    (2) INFORMATION FOR SEQ ID NO:3:

        (i) SEQUENCE CHARACTERISTICS:
            (A) LENGTH: 24 base pairs
            (B) TYPE: nucleic acid
            (C) STRANDEDNESS: single
            (D) TOPOLOGY: linear


        (xi) SEQUENCE DESCRIPTION: SEQ ID NO:3:

GACAACGGCT CCGGCATGTG CAAG                                        24

    (2) INFORMATION FOR SEQ ID NO:4:

        (i) SEQUENCE CHARACTERISTICS:
            (A) LENGTH: 24 base pairs
            (B) TYPE: nucleic acid
            (C) STRANDEDNESS: single
            (D) TOPOLOGY: linear


        (xi) SEQUENCE DESCRIPTION: SEQ ID NO:4:

TACCTGGTGC CTGGGGCGCC CCAC                                        24

```
(2) INFORMATION FOR SEQ ID NO:5:

        (i) SEQUENCE CHARACTERISTICS:
             (A) LENGTH: 25 base pairs
             (B) TYPE: nucleic acid
             (C) STRANDEDNESS: single
             (D) TOPOLOGY: linear



        (xi) SEQUENCE DESCRIPTION: SEQ ID NO:5:

GACCCGCCAA CAAGAAGGCG TACTC                                    25

(2) INFORMATION FOR SEQ ID NO:6:

        (i) SEQUENCE CHARACTERISTICS:
             (A) LENGTH: 25 base pairs
             (B) TYPE: nucleic acid
             (C) STRANDEDNESS: single
             (D) TOPOLOGY: linear



        (xi) SEQUENCE DESCRIPTION: SEQ ID NO:6:

ATGTGTACTG AGATCCCCTA TCCGT                                    25
```

## Claims

1. A process which comprises:
   a) adding a disinfectant to a sample, and
   b) lysing microorganisms in (a).

2. The process of claim 1 in which intracellular components are isolated after step (b).

3. The process of claim 2 in which DNA is isolated.

4. The process of claim 3 in which the DNA is amplified.

5. The process of claim 1 in which lysing is effected by the lysing reagent comprising proteinase K, about 100 units of achromopeptidase, and about 20 mM ethylenediamine tetraacetate.

6. The process of claim 1 in which lysing is effected by the lysing reagent achromopeptidase.

7. The process of claim 1 in which lysing is effected by heat.

8. The process of claim 1 in which the sample is liquified prior to step (a).

9. The process of claim 8 in which the sample is liquified with a solution comprising about 0.5N NaOH and about 0.1M sodium citrate containing about 1% (weight/volume) N-acetyl-L-cysteine.

10. The process of claim 1 in which the DNA isolated is selected from the group consisting of DNA from the family Legionellaceae, Neisseriacea, Enterobacteraceae, Mycobacteriacea, and Chlamydiaceae.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 520 756 (BECTON DICKINSON AND CIE) * claims 1-8,10; examples * * page 3, paragraph 3 * | 1-6,9,10 | C12N1/06 //C12N15/10 C12Q1/68 |
| X | CHEMICAL ABSTRACTS, vol. 83, no. 83, 4 August 1975, Columbus, Ohio, US; abstract no. 38196v, A.R.W. SMITH ET AL. 'DIFFERING EFFECTS OF CETYLTRIMETHYLAMMONIUMBROMIDE AND CETRIMIDE B.P. UPON GROWING CULTURES OF ESCHERICHIA COLI NCIB 8277' page 93 ;column 2 ; * abstract * & J. APPL. BACTERIOL. vol. 38, no. 2, 1975, pages 143 - 9 | 1,2 | |
| Y | CHEMICAL ABSTRACTS, vol. 111, no. 19, 6 November 1989, Columbus, Ohio, US; abstract no. 170481k, N. BOUJAFAAR ET AL. 'EXTRACTION OF CHROMOSOMAL DNA FROM STAPHYLOCOCCUS AND LISTERIA BY A RAPID ACHROMOPEPTIDASE METHOD' page 374 ;column 2 ; * abstract * & ARCH. INST. PASTEUR TUNIS vol. 65, no. 3-4, 1988, pages 271 - 8 | 1-3,5,6 | TECHNICAL FIELDS SEARCHED (Int. Cl.5) C12N |
| X Y | FR-A-1 491 224 (CANADIAN PATENTS AND DEVELOPMENT LTD) * Summary. 1; 2a,f * * page 2, column 1, paragraph 3 * * page 1, column 2, paragraph 1 * | 1-3,5,6, 9,10 1-3,6 | |
| X Y | DE-A-3 337 566 (ROHTO PHARMACEUTICAL CO) * page 6; claims 1,2; example * | 1,2 1-3,5,6 | |

-/--

The present search report has been drawn up for all claims

| Place of search | Date of completion of the search | Examiner |
|---|---|---|
| THE HAGUE | 28 APRIL 1993 | COUCKE A.O.M. |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-1 470 190 (RYSTAN COMPANY MOUNT VERNON) | 1-3,7,8 | |
| Y | * claims; examples 1,2,6,8,11,14 * <br> * page 5, paragraph 2 * <br> * page 6, paragraph 2 * <br> * page 9, paragraph 2 * <br> * page 11, paragraph 1 * <br> * page 12, paragraph 2 * <br> * page 13, paragraph 2 * <br> * page 14, paragraph 1 * <br> --- | 5,6 | |
| X | FR-A-2 043 475 (LABORATOIRES CASENNE) <br> * claims 1,5,7,9-11 * <br> * page 5 * <br> * page 8; example 1 * <br> --- | 1,2,10 | |
| X | FR-A-580 481 (M. KAHN) <br> * summary 1,2 * <br> * page 1, line 10 - page 2, line 29 * <br> --- | 1,2 | |
| A | US-A-3 962 466 (YUTAKA NAKABAYASHI) <br> * column 4, line 49 - column 5, line 23; claims; examples * | 8,9 | TECHNICAL FIELDS SEARCHED (Int. Cl.5) |

-----

The present search report has been drawn up for all claims

| Place of search | Date of completion of the search | Examiner |
|---|---|---|
| THE HAGUE | 28 APRIL 1993 | COUCKE A.O.M. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.........................................................................

& : member of the same patent family, corresponding
    document

EPO FORM 1503 03.82 (P0401)